# EUROPEAN PATENT SPECIFICATION

(11) **EP 4 463 118 B1**
(45) Date of publication and mention of the grant of the patent: **16.09.2026**
(21) Application number: 23700333.0
(22) Date of filing: 09.01.2023
(51) Int. Cl.: A61F 9/008

(54) **LASER CATARACT SURGERY USING SPIRAL LENS SEGMENTATION PATTERN**
LASERKATARAKTCHIRURGIE UNTER VERWENDUNG EINES SPIRALLINSENSEGMENTIERUNGSMUSTERS
INTERVENTION CHIRURGICALE AU LASER DE LA CATARACTE UTILISANT UN MOTIF DE SEGMENTATION DE LENTILLE EN SPIRALE

(30) Priority: 10.01.2022 US 202263266628 P
(43) Date of publication of application: 20.11.2024
(73) Proprietor: AMO Development, LLC, Irvine, CA 92618 (US)
(72) Inventor: GONZALEZ, Javier, Irvine, California 92618 (US)
(74) Representative: Carpmaels & Ransford LLP
(86) International application number: PCT/IB2023/050155
(87) International publication number: WO 2023/131916

(56) References cited:
- WO-A2-2010/075571
- DE-A1- 102019 214 020
- US-A1- 2011 196 350
- US-A1- 2014 135 749

## Description

### BACKGROUND OF THE INVENTION

### Field of the Invention

This invention relates to laser ophthalmic surgical systems, and in particular, it relates to segmentation of the crystalline lens in laser cataract surgeries.

### Description of Related Art

Eye disease can impair a patient's vision. For example, a cataract can increase the opacity of an ocular lens, and eventually, cause blindness. To restore the patient's vision, the diseased lens may be surgically removed and replaced with an artificial lens, known as an intraocular lens, or IOL. A number of medically recognized techniques are utilized for removing a cataractous lens based on, for example, phacoemulsification, mechanical cutting or destruction, laser treatments, water jet treatments, and so on.

A typical cataract surgery involves removing the eye's natural lens while leaving in place the back of the capsule which holds the lens in place. Using certain procedures, such as laser treatments along with phacoemulsification, for example, the cataract can be broken into tiny pieces that can be removed from the eye through a relatively small incision. In cataract surgery using phacoemulsification, the surgeon makes a small incision in the white portion of the eye near the outer edge of the cornea. An ultrasonic probe is then inserted through this opening and ultrasonic frequencies are used to break up the cataract into tiny pieces. The emulsified material can be simultaneously suctioned from the eye, typically using the open tip of the same instrument. To reduce the amount of ultrasonic energy used to break up the cataract, the lens can be softened and/or fragmented using a laser prior to application of ultrasonic energy. As such, the hard central core of the cataract (the nucleus) is removed first, followed by extraction of the softer, peripheral cortical fibers that make up the remainder of the lens. As compared to other forms of cataract surgery, laser-assisted cataract provides faster healing and rehabilitation as well as reduced discomfort.

Various lens segmentation patterns have been described. For example, U.S. Pat. Appl. Pub. No. 2011/0184392, entitled "Method for patterned plasma-mediated modification of the crystalline lens," describes a variety of laser treatment patterns configured to create one or more incision surfaces to segment the crystalline lens to facilitate extraction. US2011196350A1 describes a system for cataract surgery including a controller operatively coupled to a scanning optical system and configured to cause the scanning optical system to adjust a first focus position to photodisrupt a first tissue structure portion with a first laser beam, and subsequently adjust a second focus position to further photodisrupt the first tissue structure portion with a second laser beam.

### SUMMARY

The present invention is directed to an apparatus for performing a method crystalline lens segmentation that substantially obviates one or more of the problems due to limitations and disadvantages of the related art. The invention is defined by the appended independent claim. Certain embodiments are defined in the dependent claims.

An object of the present invention is to provide an apparatus for lens segmentation and extraction method in cataract surgery that reduces phacoemulsification energy required and avoids breakage of the segmented pieces during removal.

Additional features and advantages of the invention will be set forth in the descriptions that follow and in part will be apparent from the description, or may be learned by practice of the invention. The objectives and other advantages of the invention will be realized and attained by the structure particularly pointed out in the written description and claims thereof as well as the appended drawings.

To achieve the above objects, the present invention provides an ophthalmic laser surgical system for treating a patient's eye, which includes: a pulsed laser source configured to generate a pulsed laser beam; an optical delivery system including a scanner, configured to delivering a focal spot of the pulsed laser beam to the eye; and a controller connected to the laser source and the optical delivery system, configured to controls the laser source and the scanner to scan the focal spot of the pulsed laser beam within a lens of the eye according to a lens segmentation pattern to form a three-dimensional spiral volume in the lens, including to: form a boundary incision surface defining an outer boundary of the lens segmentation pattern; and form a spiral incision surface located within the outer boundary, wherein the spiral incision surface includes a plurality of horizontal steps, each horizontal step being an area located at a defined vertical position along a vertical axis, wherein any two horizontal steps that are adjacent in their vertical positions are offset in an angular direction around the vertical axis by an offset angle, and wherein a leading edge of each horizontal step is aligned with a trailing edge of its adjacent horizontal step when viewed along the vertical axis.

It is to be understood that both the foregoing general description and the following detailed description are exemplary and explanatory and are intended to provide further explanation of the invention as claimed.

### BRIEF DESCRIPTION OF THE DRAWINGS

Figure 1 schematically illustrates a spiral lens segmentation pattern according to an embodiment of the present invention.
Figure 2 is a side view of a lens of the eye that schematically illustrates the lens segmentation volume in relation to the lens capsule.
Figure 3 schematically illustrates the top views of two layers of the spiral lens segmentation pattern of Fig. 1.
Figure 4 schematically illustrates the top views of two layers of a spiral lens segmentation pattern according to a variation of the embodiment of Fig. 1.
Figure 5 schematically illustrates the top views of two layers of a spiral lens segmentation pattern according to an alternative embodiment of the present invention.
Figure 6 schematically illustrates the top views of two layers of a spiral lens segmentation pattern according to another alternative embodiment of the present invention.
Figure 7 schematically illustrates an ophthalmic laser system that may be used to implement embodiments of the present invention.

### DETAILED DESCRIPTION OF PREFERRED EMBODIMENTS

In conventional cataract extraction procedures, the laser segmentation patterns break the lens into chunks. These chunks, however, typically cannot be extracted from the lens capsule in whole pieces, requiring additional phacoemulsification energy to break the individual chunks into smaller pieces. For instance, in one exemplary method, the lens is cut into quadrants with an additional softening pattern, which breaks the lens into vertical rectangular columns that extend the entire depth of the segmentation region. The individual rectangular blocks cannot be extracted without farther subdivisions due the large surface area that connects each block to the other blocks or the rest of the lens. Additional phacoemulsification energy is required to subdivide the blocks.

Embodiments of the present invention provide a new geometry of a lens segmentation pattern that reduces the phacoemulsification energy required to remove the lens. The lens segmentation process employs a three-dimensional spiral lens segmentation pattern that resembles a spiral staircase or a spiral ramp, to incise a vertical cylindrical volume of the lens into a three-dimensional spiral that can be more easily removed.

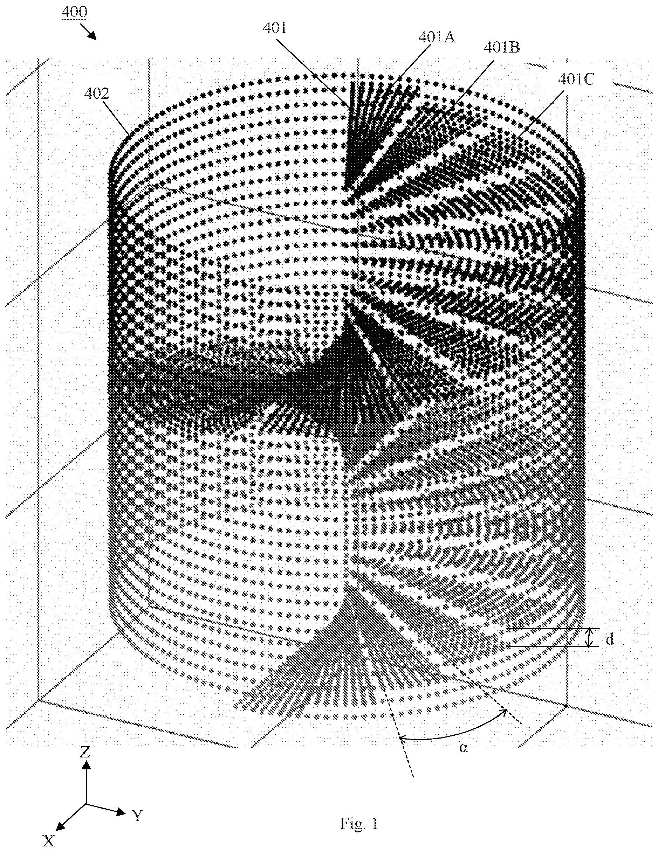

In a preferred embodiment schematically illustrated in Fig. 1, the spiral incision surface 401 that creates the three-dimensional spiral is shaped like a spiral staircase, formed by a series of offset horizontal (X-Y plane) incisions 401A, 401B, 401C, etc., referred to as "horizontal steps" in this disclosure for convenience. Adjacent horizontal steps are offset in the vertical, i.e. depth, direction (the Z-axis direction) by a step height, and in the angular direction (around the Z axis) by an offset angle. In the illustrated embodiment, each horizontal step 401A, 401B, 401C etc. has the shape of a sector of a circle, which is a region inside a circle bounded by two radii and an arc. The offset angle between any two adjacent horizontal steps is such that the leading edge of one horizontal step and the trailing edge of the other horizontal step are aligned when viewed in the Z direction.

The spiral incision surface 401 may be described as being formed by a radius of a circle centered on the Z axis, starting at a defined Z position and a defined angular position, first rotating around the Z axis by a defined angular amount (offset angle), then moving along the Z axis for a step height, then rotating around the Z axis again by another offset angle, then moving along the Z axis again for another step height, etc. All rotations are in the same rotation direction and all movements along the Z axis are in the same Z direction.

In a preferred embodiment, the horizontal steps have equal angular sizes, and the offset angles between adjacent horizontal steps are equal to each other throughout the spiral incision and are equal to the angular size of the horizontal steps. In a preferred embodiment, the step heights between adjacent horizontal steps are equal to each other throughout the spiral incision. In alternative embodiments, the offset angles and step heights may be variable throughout the spiral incision. In another preferred embodiment, there are no steps, but the incision forms a continuous ramp of constant or variable slope.

The lens segmentation pattern also includes a cylindrical incision surface 402 that defines the outer boundary of the spiral segmentation volume 400. The pattern may also include a top incision and/or a bottom incision respectively defining the top and/or bottom boundaries of the cylindrical spiral segmentation volume. The top and/or bottom incisions may be flat (horizontal), or curved (e.g., convex). Alternatively, the top and/or bottom of the spiral segmentation volume may be defined by the top and/or bottom surface of the lens itself.

The spiral incision surface 401 and the cylindrical incision surface 402 incise the cylindrical volume 400 into a three-dimensional spiral volume, similar to a "curly fry," although a curly fry is typically hollow at the cylindrical center while the three-dimensional spiral volume in Fig. 1 extends fully to the center axis of the cylinder. The three-dimensional spiral volume has a width equal to the diameter of the cylinder, a slice thickness D (the vertical distance between two steps that are separated by 2π in angular position) equal to *D=(2π*/*α)*d,* where *α* is the offset angle between adjacent horizontal steps and *d* is the step height. The number of turns of the three-dimensional spiral is equal to *N*=*T*/*D,* where *T* is the total height of the cylinder in the Z direction. Note that the above equations apply to the situation when all offset angles are identical and all step heights are identical.

When removing the three-dimensional spiral volume of tissue from the eye, the three-dimensional spiral is pulled at the top end and removed (suctioned into the open tip of the phacoemulsification tool) in a continuous manner as the spiral unwinds. At any given moment in time, only a given section of the spiral is being separated and detached from its surrounding lens tissue, while the remaining parts of the spiral stay unaffected. Therefore, only the section currently being separated experiences a severing force at its incision surfaces. Thus, the required pulling force applied to the spiral is relatively small. Consequently, breakage of the spiral is avoided or minimized, and the spiral can be pulled out in one piece or a small number of pieces.

In contrast, in the conventional technology where the lens is segmented into multiple vertical columns, in order to pull an individual column up, the entire column must detach from the surrounding tissue at the same time and be displaced relative to the surrounding tissue along its entire length. Therefore, the severing force experienced by the entire column is much larger as compared to the force needed to pull the spiral. This requires a large pulling force applied to the top of the column, which often causes the column to break. In practice, it is difficult to pull the entire column out in one piece. As a result, each column typically needs to be removed bit by bit, which is time consuming. As mentioned earlier, using the spiral segmentation according to embodiments of the present invention, the spiral may still break, but lest often than in the conventional technology. The inventor believes that the reasons are as follows.

The process can be casted in terms of energy release rate, which is a concept in fracture mechanics. The energy release rate (G) is the energy (potential) per additional surface area in the fracture surface. In order to take out the entire column as in the conventional technology, one needs to create a fracture surface that extends the entire depth of the column (total surface area A = 2*(width+length)*depth). Note that the column is elastic, so the above A is an upper bound. In reality, an upper portion of the column stretches, so as to be displaced enough from the rest of the lens, that its sides locally fracture. If the lens is very elastic, the stretch is larger, and the fractured portion has smaller depth. In the case the column of lens is perfectly rigid, the entire column needs to be detached at once, over a fracture area of A.

In the case of the spiral segmentation according to embodiments of the present invention, the extracted portion of the lens is not axially stretching, but locally bending, and hence the local displacements (with respect to the matrix or rest of the lens) are larger and localized. The energy used to fracture the staircase is directed over a smaller fracture surface, then the energy release rate can be maintained by pulling the staircase from the top (or at least, one of the small number of chuck that are pulled out, which is much larger than the chunks pulled out using the conventional technology).

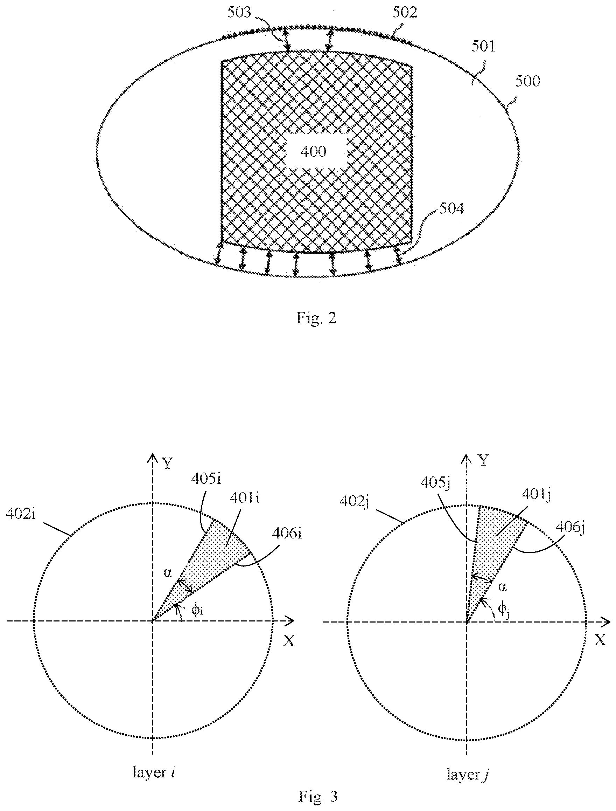

Note that the cylindrical lens segmentation region 400 only corresponds to a center portion of the crystalline lens 500, which is the hard central core of the cataract, as schematically illustrated in Fig. 2. During the cataract procedure, after the cylindrical region 400 is removed as described above, the remaining softer, peripheral portion 501 of the lens is then removed in a conventional manner. In practice, the cylindrical lens segmentation region 400 is typically about 5 mm diameter, or approximately as wide as the iris. The entire lens 500 is approximately twice as wide as the cylindrical lens segmentation region 400.

Fig. 2 also illustrates an anterior capsular opening 502, an anterior safety zone (an uncut zone) 503 which is left between the lens segmentation region 400 and the anterior lens capsule, and a posterior safety zone 504 which is left between the lens segmentation region 400 and the posterior lens capsule. The anterior and posterior safety zones are optional; when employed, they serve to prevent accidental cutting of the lens capsule.

In a preferred embodiment, the lens segmentation pattern including spiral incision surface 401 and cylindrical incision surface 402 is formed by scanning a focal spot of a pulsed laser beam in the lens tissue one layer at a time, preferably from bottom to top. A layer is a two-dimensional laser scan pattern located in a horizontal (X-Y) plane at a defined depth (Z value). For each layer *i*, as schematically illustrated in Fig. 3 (top view), the laser focal spot is scanned to form a circle 402i which is a cross-section of the cylinder 402 at that depth, and to form a filled sector (the horizontal step) 401i of the circle. The sector 4011 may be formed by scanning the laser focal spot along a plurality of radial lines (see Fig. 1), or along a plurality of concentric arcs, or using other suitable scan patterns.

Here, it should be noted that in practice, it may not be possible to form a perfectly flat horizontal plane in the scan, for reasons related to how the scanner device is implemented (e.g., with two scanning mirrors, described in more detail later). In this disclosure, a "layer" or a "horizontal plane" should be understood to mean a scan pattern where the Z parameter is set as a constant by the controller, even though the actual Z values of the resulting laser focal spots within the layer or plane may vary slightly.

Fig. 3 also shows a layer *j* located immediately above layer *i*. The trailing edge 406j of the horizontal step 401j is vertically aligned with the leading edge 405i of the horizontal step 401i immediately below it. Note that in this disclosure, vertically "aligned" means that when viewed along the Z direction, the two edges coincide with each other or are separated by no more than the average spot-to-spot distance between laser focal spots within each layer. The angular position *ϕⱼ* of the sector 401j of layer *j* is offset from the angular position *ϕᵢ* of the sector 401i of layer *i* by the offset angle *α*: *ϕⱼ* = *ϕᵢ* + *α.* The depth value *Zⱼ* of layer *j* is offset from the depth value *Zᵢ* of layer *i* by the step height *d: Zⱼ* = *Zᵢ + d.* Note that the angular positions of the sectors may be defined at any predefined location of the sector, such as the trailing edge 406i in this example, or (not shown) the leading edge 405i, the center radius, or another position.

When all offset angles are equal in size and all step heights are equal in size, for a given layer *i,* the depth value of layer *i* is *Zᵢ* = *Z₀ + i*d,* where *Z₀* is a constant, and the angular position *ϕᵢ* of the sector 4011 is *ϕᵢ* = *ϕ₀ + i*α,* where *ϕ₀* is a constant. *Zᵢ* can also be written as a function of *ϕᵢ*: *Zᵢ* = *Z₀* + *(ϕᵢ - ϕ₀)***d*/*α.*

In preferred embodiments, the step height d is sufficiently small, e.g., approximately the same as the average spot-to-spot distance between laser focal spots within each layer (e.g., the step height is between 67% and 150% of the average spot-to-spot distance), so that the circles 402i of all layers collectively form the cylindrical incision surface 402 that defines the outer boundary of the lens segmentation volume. Likewise, due to the small step height, the vertical area between the leading edge of one horizontal step and the trailing edge of the adjacent horizontal step does not form a gap, but constitutes a part of the overall spiral incision surface 401.

If the bottom and/or top incisions of the cylindrical volume are required, they may be formed before and after the other layers, respectively.

Although less preferred, it is also possible to form the entire outer cylindrical surface 402 at once, before or after forming the spiral incision surface 401.

Many variations of the geometry of the spiral incision surface 401 may be employed. For example, while the spiral incision surface 401 shown in Fig. 1 is a right hand spiral, a left hand spiral may alternatively be used. Although in the illustrated embodiment the horizontal steps have equal angular sizes and equal step heights, they may alternatively have unequal angular sizes and/or unequal step heights. It is also possible to form intermediate layers between two adjacent layers that have horizontal steps, where the intermediate layer includes only a circular line and a single radial line located on the vertical face between the two adjacent horizontal steps.

Further, many variations of the geometry of the outer boundary incision surface 402 may be employed. For example, although in the embodiment of Figs. 1 and 3 the cross-sectional shape of the cylinder is a circle, it may alternatively be an oval or other shapes. The outer boundary of the segmentation volume may even have different cross-sectional sizes and/or shapes at different depths; e.g., it may be a tapered cylinder, a truncated cone, a barrel shape, etc. In such cases, when forming each layer, the outer circle 402i in Fig. 3 will be replaced by a closed curve having a defined shape corresponding to the shape of the outer boundary.

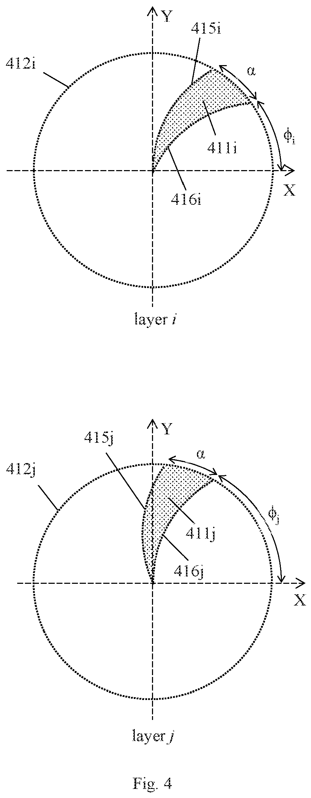

Another variation of the spiral segmentation pattern is illustrated in Fig. 4 (top view). Unlike the horizontal steps 401i in the embodiment of Figs. 1 and 3 which are sectors of the circle, the horizontal step 411i in the embodiment of Fig. 4 has the shape of a modified sector of the circle 412i, bound by two curves 415i and 416i and an arc. The two curves 415i and 416i are identical in shape but are rotated with respect to each other by an offset angle *α.* The horizontal step 411j of the immediately adjacent layer *j* has the same shape as the horizontal step 411i of layer *i* but is rotated by the offset angle *α.* The leading edge 415i of the horizontal step 411i is vertically aligned with the trailing edge 416j of the horizontal step 411j. The embodiment of Fig. 4 is otherwise similar to the embodiment of Figs. 1 and 3. The three-dimensional spiral incision surface in this variation may be described as being formed by a curve that extends from the Z axis perpendicularly, rotating around and moving along the Z axis in a similar manner as described earlier for the embodiment of Figs. 1 and 3.

In further variations, the leading edge and the trailing edge of each horizontal step may have different shapes, as long as the leading edge of one horizontal step is vertically aligned with the trailing edge of the adjacent horizontal step.

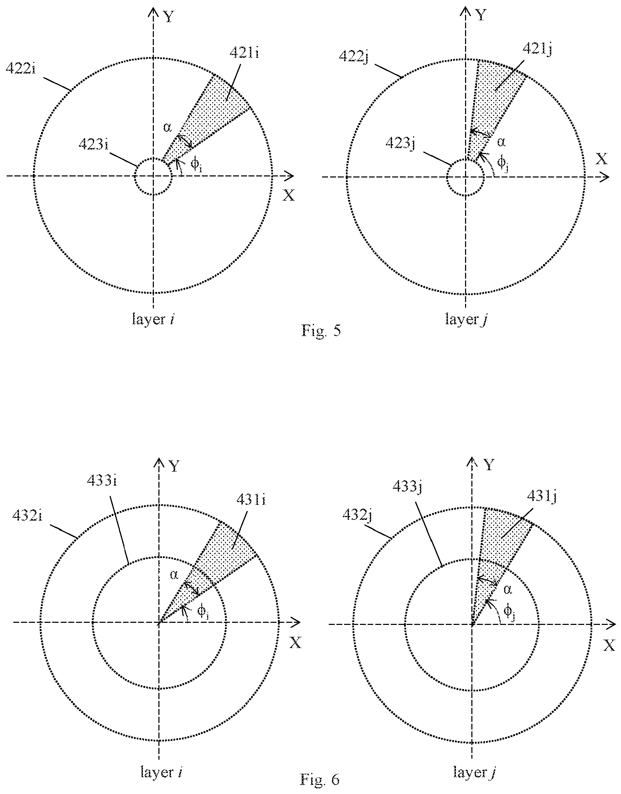

In an alternative embodiment, schematically illustrated in Fig. 5, a small, inner cylinder is formed concentrically with the outer cylinder, and each horizontal step extends between the inner cylinder and the outer cylinder, rather than reaching the Z axis. As shown in Fig. 5 (top view), for each layer *i*, the laser focal spot is scanned to form an outer circle 422i that corresponds to the outer cylinder, as well as a smaller, concentric inner circle 423i that corresponds to the inner cylinder. The laser focal spots also form a truncated sector (the horizontal step) 421i which is bound by two radial lines and two arcs respectively on the outer and inner circles. Fig. 5 also shows the adjacent layer *j* located immediately above layer *i*, which includes an outer circle 422j, an inner circle 423j, and a truncated sector 421j. The inner circles 423i, 423j, etc. collectively form the incision surface that define the inner cylinder. Other aspects of this alternative embodiment are similar to those of the embodiment of Figs. 1 and 3.

In the embodiment of Fig. 5, the three-dimensional spiral volume formed by the two cylindrical incision and the spiral incision is similar to a "curly fry." The three-dimensional spiral has a width equal to the radial distance between the inner and outer cylinders. The three-dimensional spiral volume formed in this embodiment may be removed from the eye in a similar manner as the spiral volume formed in the embodiment of Figs. 1 and 3. After the spiral volume is removed, the small inner cylinder may be removed.

In another alternative embodiment, schematically illustrated in Fig. 6, the cylindrical volume is incised into two nested spiral volumes. As shown in Fig. 6 (top view), for each layer *i*, the laser focal spot is scanned to form an outer circle 432i that corresponds to the outer cylinder, as well as an intermediate circle 433i that is smaller than and concentric with the outer circle. The laser focal spots also form a filled sector (the horizontal step) 431i of the outer circle 432i. Fig. 6 also shows the adjacent layer *j* located immediately above layer *i*, which includes an outer circle 432j, an intermediate circle 433j, and a horizontal step 431j. The intermediate circles 433i, 433j, etc. collectively form an intermediate cylindrical incision that serves to divide the two nested spiral volumes. Other aspects of this alternative embodiment are similar to those of the embodiment of Figs. 1 and 3. The two nested spiral volumes may be removed from the eye separately, with the inner spiral volume removed first or second. Moreover, the spirals may or may not be in the same direction.

More than two nested spiral volumes may be formed by using additional intermediate cylindrical incisions. The widths of two or more nested spiral volumes may be adjusted by adjusting the radial positions of the intermediate cylindrical incisions. The two or more nested spirals may be independent of each other and have different offset angles between adjacent steps. Further, the pattens of Fig. 5 and Fig. 6 may be combined, e.g., to form an inner cylinder and two nested spirals.

The variations described above in connection with Figs. 1 and 3 also apply to the alternative embodiments of Figs. 4, 5 and 6.

As can be seen from the above descriptions, the lens segmentation pattern according to embodiments of the present invention creates one, two or more chunks in the lens, each having a spiral geometry that allows it to be extracted as a single piece by unwinding. Each chuck is relatively large to be structurally strong enough to be extracted as a whole piece (or a relatively small number of pieces in comparison with the column in the conventional technology), preventing breakage while pulling. This technique substantially reduces the phacoemulsification energy required for lens removal, and reduces the total amount of time required for the removal.

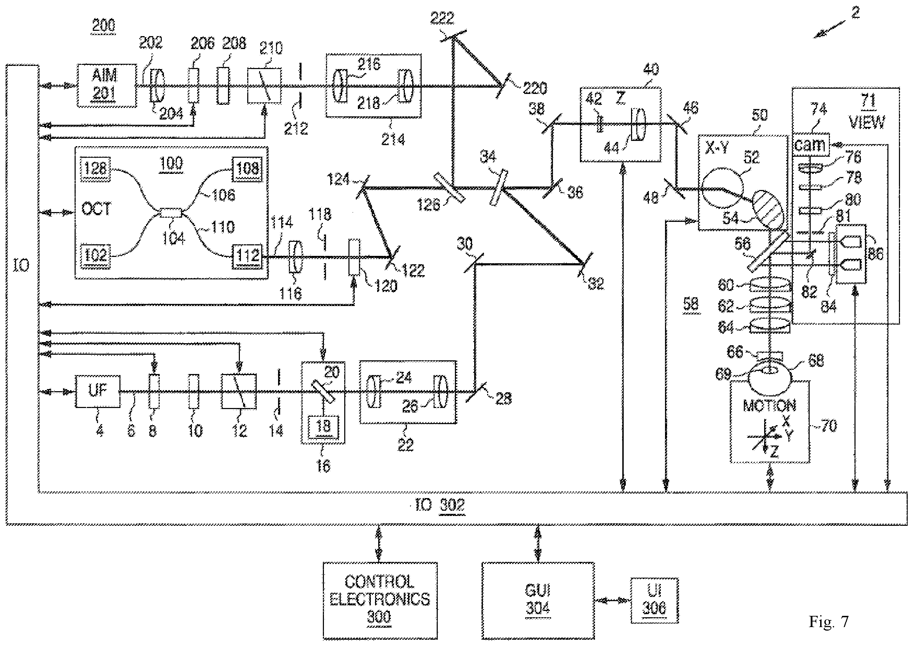

The above described embodiments may be implemented using any suitable ophthalmic laser system. Such a laser system typically includes a pulsed laser source capable of generating a pulsed laser beam, and an optical delivery system including focusing lenses and scanners for delivering a focal spot of the pulsed laser beam to the eye tissue. The scanners may include an XY scanner and a Z scanner. A controller is connected to and controls the laser source and the scanners to scan the laser focal spot according to preprogrammed scan patterns described above. An exemplary laser system is shown in Fig. 7.

The ophthalmic laser system 2 shown in Fig. 7 includes an ultrafast (UF) light source 4 (e.g. a femtosecond laser). Using this system, a beam may be scanned in a patient's eye in three dimensions: X, Y, Z. In this embodiment, the UF wavelength can vary between 1010 nm to 1100 nm and the pulse width can vary from 100 fs to 10000 fs. The pulse repetition frequency can also vary from 10 kHz to 250 kHz. Safety limits with regard to unintended damage to non-targeted tissue bound the upper limit with regard to repetition rate and pulse energy; while threshold energy, time to complete the procedure and stability bound the lower limit for pulse energy and repetition rate. The peak power of the focused spot in the eye 68 and specifically within the crystalline lens 69 and anterior capsule of the eye is sufficient to produce optical breakdown and initiate a plasma-mediated ablation process. Near-infrared wavelengths are preferred because linear optical absorption and scattering in biological tissue is reduced across that spectral range. As an example, laser 4 may be a repetitively pulsed 1035 nm device that produces 500 fs pulses at a repetition rate of 100 kHz and an individual pulse energy in the ten microjoule range.

The laser 4 is controlled by control electronics 300, via an input and output device 302, to create optical beam 6. Control electronics 300 may be a computer, microcontroller, etc. In this example, the entire system is controlled by the controller 300, and data moved through input/output device IO 302. A graphical user interface GUI 304 may be used to set system operating parameters, process user input (UI) 306 on the GUI 304, and display gathered information such as images of ocular structures.

The generated UF light beam 6 proceeds towards the patient eye 68 passing through half-wave plate, 8, and linear polarizer, 10. The polarization state of the beam can be adjusted so that the desired amount of light passes through half-wave plate 8 and linear polarizer 10, which together act as a variable attenuator for the UF beam 6. Additionally, the orientation of linear polarizer 10 determines the incident polarization state incident upon beamcombiner 34, thereby optimizing beamcombiner throughput.

The UF beam proceeds through a shutter 12, aperture 14, and a pickoff device 16. The system controlled shutter 12 ensures on/off control of the laser for procedural and safety reasons. The aperture sets an outer useful diameter for the laser beam and the pickoff monitors the output of the useful beam. The pickoff device 16 includes of a partially reflecting mirror 20 and a detector 18. Pulse energy, average power, or a combination may be measured using detector 18. The information can be used for feedback to the half-wave plate 8 for attenuation and to verify whether the shutter 12 is open or closed. In addition, the shutter 12 may have position sensors to provide a redundant state detection.

The beam passes through a beam conditioning stage 22, in which beam parameters such as beam diameter, divergence, circularity, and astigmatism can be modified. In this illustrative example, the beam conditioning stage 22 includes a 2 element beam expanding telescope comprised of spherical optics 24 and 26 in order to achieve the intended beam size and collimation. Although not illustrated here, an anamorphic or other optical system can be used to achieve the desired beam parameters. The factors used to determine these beam parameters include the output beam parameters of the laser, the overall magnification of the system, and the desired numerical aperture (NA) at the treatment location. In addition, the optical system 22 can be used to image aperture 14 to a desired location (e.g. the center location between the 2-axis scanning device 50 described below). In this way, the amount of light that makes it through the aperture 14 is assured to make it through the scanning system. Pickoff device 16 is then a reliable measure of the usable light.

After exiting conditioning stage 22, beam 6 reflects off of fold mirrors 28, 30, and 32. These mirrors can be adjustable for alignment purposes. The beam 6 is then incident upon beam combiner 34. Beamcombiner 34 reflects the UF beam 6 (and transmits both the OCT 114 and aim 202 beams described below). For efficient beamcombiner operation, the angle of incidence is preferably kept below 45 degrees and the polarization where possible of the beams is fixed. For the UF beam 6, the orientation of linear polarizer 10 provides fixed polarization.

Following the beam combiner 34, the beam 6 continues onto the z-adjust or Z scan device 40. In this illustrative example the z-adjust includes a Galilean telescope with two lens groups 42 and 44 (each lens group includes one or more lenses). Lens group 42 moves along the z-axis about the collimation position of the telescope. In this way, the focus position of the spot in the patient's eye 68 moves along the z-axis as indicated. In general there is a fixed linear relationship between the motion of lens 42 and the motion of the focus. In this case, the z-adjust telescope has an approximate 2x beam expansion ratio and a 1:1 relationship of the movement of lens 42 to the movement of the focus. Alternatively, lens group 44 could be moved along the z-axis to actuate the z-adjust, and scan. The z-adjust is the z-scan device for treatment in the eye 68. It can be controlled automatically and dynamically by the system and selected to be independent or to interplay with the X-Y scan device described next. Mirrors 36 and 38 can be used for aligning the optical axis with the axis of z-adjust device 40.

After passing through the z-adjust device 40, the beam 6 is directed to the x-y scan device by mirrors 46 and 48. Mirrors 46 and 48 can be adjustable for alignment purposes. X-Y scanning is achieved by the scanning device 50 preferably using two mirrors 52 and 54 under the control of control electronics 300, which rotate in orthogonal directions using motors, galvanometers, or any other well known optic moving device. Mirrors 52 and 54 are located near the telecentric position of the objective lens 58 and contact lens 66 combination described below. Tilting these mirrors 52/54 causes them to deflect beam 6, causing lateral displacements in the plane of UF focus located in the patient's eye 68. Objective lens 58 may be a complex multi-element lens element, as shown, and represented by lenses 60, 62, and 64. The complexity of the lens 58 will be dictated by the scan field size, the focused spot size, the available working distance on both the proximal and distal sides of objective 58, as well as the amount of aberration control. An f-theta lens 58 of focal length 60 mm generating a spot size of 10 µm, over a field of 10 mm, with an input beam size of 15 mm diameter is an example. Alternatively, X-Y scanning by scanner 50 may be achieved by using one or more moveable optical elements (e.g. lenses, gratings) which also may be controlled by control electronics 300, via input and output device 302.

The aiming and treatment scan patterns can be automatically generated by the scanner 50 under the control of controller 300. Such patterns may be comprised of a single spot of light, multiple spots of light, a continuous pattern of light, multiple continuous patterns of light, and/or any combination of these. In addition, the aiming pattern (using aim beam 202 described below) need not be identical to the treatment pattern (using light beam 6), but preferably at least defines its boundaries in order to assure that the treatment light is delivered only within the desired target area for patient safety. This may be done, for example, by having the aiming pattern provide an outline of the intended treatment pattern. This way the spatial extent of the treatment pattern may be made known to the user, if not the exact locations of the individual spots themselves, and the scanning thus optimized for speed, efficiency and accuracy. The aiming pattern may also be made to be perceived as blinking in order to further enhance its visibility to the user.

An optional contact lens 66, which can be any suitable ophthalmic lens, can be used to help further focus the optical beam 6 into the patient's eye 68 while helping to stabilize eye position. The positioning and character of optical beam 6 and/or the scan pattern the beam 6 forms on the eye 68 may be further controlled by use of an input device such as a joystick, or any other appropriate user input device (e.g. GUI 304) to position the patient and/or the optical system.

The UF laser 4 and controller 300 can be set to target the surfaces of the targeted structures in the eye 68 and ensure that the beam 6 will be focused where appropriate and not unintentionally damage non-targeted tissue. Imaging modalities and techniques described herein, such as for example, Optical Coherence Tomography (OCT), Purkinje imaging, Scheimpflug imaging, or ultrasound may be used to determine the location and measure the thickness of the lens and lens capsule to provide greater precision to the laser focusing methods, including 2D and 3D patterning. Laser focusing may also be accomplished using one or more methods including direct observation of an aiming beam, Optical Coherence Tomography (OCT), Purkinje imaging, Scheimpflug imaging, ultrasound, or other known ophthalmic or medical imaging modalities and/or combinations thereof. In the system shown in Fig. 7, an OCT device 100 is described, although other modalities are within the scope of the present invention. An OCT scan of the eye will provide information about the axial location of the anterior and posterior lens capsule, the boundaries of the cataract nucleus, as well as the depth of the anterior chamber. This information is then be loaded into the control electronics 300, and used to program and control the subsequent laser-assisted surgical procedure. The information may also be used to determine a wide variety of parameters related to the procedure such as, for example, the upper and lower axial limits of the focal planes used for cutting the lens capsule and segmentation of the lens cortex and nucleus, and the thickness of the lens capsule among others.

The OCT device 100 in Fig. 7 includes a broadband or a swept light source 102 that is split by a fiber coupler 104 into a reference arm 106 and a sample arm 110. The reference arm 106 includes a module 108 containing a reference reflection along with suitable dispersion and path length compensation. The sample arm 110 of the OCT device 100 has an output connector 112 that serves as an interface to the rest of the UF laser system. The return signals from both the reference and sample arms 106, 110 are then directed by coupler 104 to a detection device 128, which employs either time domain, frequency or single point detection techniques. In Fig. 7, a frequency domain technique is used with an OCT wavelength of 920 nm and bandwidth of 100 nm.

Exiting connector 112, the OCT beam 114 is collimated using lens 116. The size of the collimated beam 114 is determined by the focal length of lens 116. The size of the beam 114 is dictated by the desired NA at the focus in the eye and the magnification of the beam train leading to the eye 68. Generally, OCT beam 114 does not require as high an NA as the UF beam 6 in the focal plane and therefore the OCT beam 114 is smaller in diameter than the UF beam 6 at the beamcombiner 34 location. Following collimating lens 116 is aperture 118 which further modifies the resultant NA of the OCT beam 114 at the eye. The diameter of aperture 118 is chosen to optimize OCT light incident on the target tissue and the strength of the return signal. Polarization control element 120, which may be active or dynamic, is used to compensate for polarization state changes which may be induced by individual differences in corneal birefringence, for example. Mirrors 122 and 124 are then used to direct the OCT beam 114 towards beam combiners 126 and 34. Mirrors 122 and 124 may be adjustable for alignment purposes and in particular for overlaying of OCT beam 114 to UF beam 6 subsequent to beamcombiner 34. Similarly, beamcombiner 126 is used to combine the OCT beam 114 with the aim beam 202 described below.

Once combined with the UF beam 6 subsequent to beamcombiner 34, OCT beam 114 follows the same path as UF beam 6 through the rest of the system. In this way, OCT beam 114 is indicative of the location of UF beam 6. OCT beam 114 passes through the z-scan 40 and x-y scan 50 devices then the objective lens 58, contact lens 66 and on into the eye 68. Reflections and scatter off of structures within the eye provide return beams that retrace back through the optical system, into connector 112, through coupler 104, and to OCT detector 128. These return back reflections provide the OCT signals that are in turn interpreted by the system as to the location in X, Y Z of UF beam 6 focal location.

OCT device 100 works on the principle of measuring differences in optical path length between its reference and sample arms. Therefore, passing the OCT through z-adjust 40 does not extend the z-range of OCT system 100 because the optical path length does not change as a function of movement of 42. OCT system 100 has an inherent z-range that is related to the detection scheme, and in the case of frequency domain detection it is specifically related to the spectrometer and the location of the reference arm 106. In the case of OCT system 100 used in Fig. 7, the z-range is approximately 1-2 mm in an aqueous environment. Extending this range to at least 4 mm involves the adjustment of the path length of the reference arm within OCT system 100. Passing the OCT beam 114 in the sample arm through the z-scan of z-adjust 40 allows for optimization of the OCT signal strength. This is accomplished by focusing the OCT beam 114 onto the targeted structure while accommodating the extended optical path length by commensurately increasing the path within the reference arm 106 of OCT system 100.

Because of the fundamental differences in the OCT measurement with respect to the UF focus device due to influences such as immersion index, refraction, and aberration, both chromatic and monochromatic, care must be taken in analyzing the OCT signal with respect to the UF beam focal location. A calibration or registration procedure as a function of X, Y Z should be conducted in order to match the OCT signal information to the UF focus location and also to the relate to absolute dimensional quantities. Observation of an aim beam may also be used to assist the user to directing the UF laser focus. Additionally, an aim beam visible to the unaided eye in lieu of the infrared OCT and UF beams can be helpful with alignment provided the aim beam accurately represents the infrared beam parameters. An aim subsystem 200 is employed in the configuration shown in Fig. 7. The aim beam 202 is generated by a an aim beam light source 201, such as a helium-neon laser operating at a wavelength of 633 nm. Alternatively a laser diode in the 630-650 nm range could be used. The advantage of using the helium neon 633 nm beam is its long coherence length, which would enable the use of the aim path as a laser unequal path interferometer (LUPI) to measure the optical quality of the beam train, for example.

Once the aim beam light source generates aim beam 202, the aim beam 202 is collimated using lens 204. The size of the collimated beam is determined by the focal length of lens 204. The size of the aim beam 202 is dictated by the desired NA at the focus in the eye and the magnification of the beam train leading to the eye 68. Generally, aim beam 202 should have close to the same NA as UF beam 6 in the focal plane and therefore aim beam 202 is of similar diameter to the UF beam at the beamcombiner 34 location. Because the aim beam is meant to stand-in for the UF beam 6 during system alignment to the target tissue of the eye, much of the aim path mimics the UF path as described previously. The aim beam 202 proceeds through a half-wave plate 206 and linear polarizer 208. The polarization state of the aim beam 202 can be adjusted so that the desired amount of light passes through polarizer 208. Elements 206 and 208 therefore act as a variable attenuator for the aim beam 202. Additionally, the orientation of polarizer 208 determines the incident polarization state incident upon beamcombiners 126 and 34, thereby fixing the polarization state and allowing for optimization of the beamcombiners' throughput. Of course, if a semiconductor laser is used as aim beam light source 200, the drive current can be varied to adjust the optical power.

The aim beam 202 proceeds through a shutter 210 and aperture 212. The system controlled shutter 210 provides on/off control of the aim beam 202. The aperture 212 sets an outer useful diameter for the aim beam 202 and can be adjusted appropriately. A calibration procedure measuring the output of the aim beam 202 at the eye can be used to set the attenuation of aim beam 202 via control of polarizer 206.

The aim beam 202 next passes through a beam conditioning device 214. Beam parameters such as beam diameter, divergence, circularity, and astigmatism can be modified using one or more well known beaming conditioning optical elements. In the case of an aim beam 202 emerging from an optical fiber, the beam conditioning device 214 can simply include a beam expanding telescope with two optical elements 216 and 218 in order to achieve the intended beam size and collimation. The final factors used to determine the aim beam parameters such as degree of collimation are dictated by what is necessary to match the UF beam 6 and aim beam 202 at the location of the eye 68. Chromatic differences can be taken into account by appropriate adjustments of beam conditioning device 214. In addition, the optical system 214 is used to image aperture 212 to a desired location such as a conjugate location of aperture 14.

The aim beam 202 next reflects off of fold mirrors 222 and 220, which are preferably adjustable for alignment registration to UF beam 6 subsequent to beam combiner 34. The aim beam 202 is then incident upon beam combiner 126 where the aim beam 202 is combined with OCT beam 114. Beamcombiner 126 reflects the aim beam 202 and transmits the OCT beam 114, which allows for efficient operation of the beamcombining functions at both wavelength ranges. Alternatively, the transmit and reflect functions of beamcombiner 126 can be reversed and the configuration inverted. Subsequent to beamcombiner 126, aim beam 202 along with OCT beam 114 is combined with UF beam 6 by beamcombiner 34.

A device for imaging the target tissue on or within the eye 68 is shown schematically in Fig. 7 as imaging system 71. Imaging system includes a camera 74 and an illumination light source 86 for creating an image of the target tissue. The imaging system 71 gathers images which may be used by the system controller 300 for providing pattern centering about or within a predefined structure. The illumination light source 86 for the viewing is generally broadband and incoherent. For example, light source 86 can include multiple LEDs as shown. The wavelength of the viewing light source 86 is preferably in the range of 700 nm to 750 nm, but can be anything which is accommodated by the beamcombiner 56, which combines the viewing light with the beam path for UF beam 6 and aim beam 202 (beamcombiner 56 reflects the viewing wavelengths while transmitting the OCT and UF wavelengths). The beamcombiner 56 may partially transmit the aim wavelength so that the aim beam 202 can be visible to the viewing camera 74. Optional polarization element 84 in front of light source 86 can be a linear polarizer, a quarter wave plate, a half-wave plate or any combination, and is used to optimize signal. A false color image as generated by the near infrared wavelength is acceptable.

The illumination light from light source 86 is directed down towards the eye using the same objective lens 58 and contact lens 66 as the UF and aim beam 6, 202. The light reflected and scattered off of various structures in the eye 68 are collected by the same lenses 58 and 66 and directed back towards beamcombiner 56. There, the return light is directed back into the viewing path via beam combiner and mirror 82, and on to camera 74. Camera 74 can be, for example but not limited to, any silicon based detector array of the appropriately sized format. Video lens 76 forms an image onto the camera's detector array while optical elements 80 and 78 provide polarization control and wavelength filtering respectively. Aperture or iris 81 provides control of imaging NA and therefore depth of focus and depth of field. A small aperture provides the advantage of large depth of field which aids in the patient docking procedure. Alternatively, the illumination and camera paths can be switched. Furthermore, aim light source 200 can be made to emit in the infrared which would not directly visible, but could be captured and displayed using imaging system 71.

Coarse adjust registration is usually needed so that when the contact lens 66 comes into contact with the cornea, the targeted structures are in the capture range of the X, Y scan of the system. Therefore a docking procedure is preferred, which preferably takes in account patient motion as the system approaches the contact condition (i.e. contact between the patient's eye 68 and the contact lens 66. The viewing system 71 is configured so that the depth of focus is large enough such that the patient's eye 68 and other salient features may be seen before the contact lens 66 makes contact with eye 68.

Preferably, a motion control system 70 is integrated into the overall control system 2, and may move the patient, the system 2 or elements thereof, or both, to achieve accurate and reliable contact between contact lens 66 and eye 68. Furthermore, a vacuum suction subsystem and flange may be incorporated into system 2, and used to stabilize eye 68. The alignment of eye 68 to system 2 via contact lens 66 may be accomplished while monitoring the output of imaging system 71, and performed manually or automatically by analyzing the images produced by imaging system 71 electronically by means of control electronics 300 via IO 302. Force and/or pressure sensor feedback may also be used to discern contact, as well as to initiate the vacuum subsystem.

It will be apparent to those skilled in the art that various modification and variations can be made in the lens segmentation apparatus of the present invention without departing from the scope of the invention. Thus, it is intended that the present invention cover modifications and variations that come within the scope of the appended claims.

## Claims

1. An ophthalmic laser surgical system for treating a patient's eye, comprising:
a pulsed laser source (4) configured to generate a pulsed laser beam (6);
an optical delivery system including a scanner (50), configured to delivering a focal spot of the pulsed laser beam to the eye (68); and
a controller (300) connected to the laser source and the optical delivery system, configured to controls the laser source and the scanner to scan the focal spot of the pulsed laser beam within a lens (500) of the eye according to a lens segmentation pattern to form a three-dimensional spiral volume in the lens, including to:
form a boundary incision surface (402) defining an outer boundary of the lens segmentation pattern; and
form a spiral incision surface (401) located within the outer boundary,
wherein the spiral incision surface includes a plurality of horizontal steps (401A-403C, 401i-401j, 411i-j, 421i-421j, 431i-431j, ), each horizontal step being an area located at a defined vertical position along a vertical axis, wherein any two horizontal steps that are adjacent in their vertical positions are offset in an angular direction around the vertical axis by an offset angle (α), and wherein a leading edge of each horizontal step is aligned with a trailing edge of its adjacent horizontal step when viewed along the vertical axis.

2. The ophthalmic laser surgical system of claim 1, wherein the boundary incision surface is a cylindrical surface.

3. The ophthalmic laser surgical system of claim 2, wherein the boundary incision surface is a round cylindrical surface, and wherein each horizontal step has a shape of a sector of a circle.

4. The ophthalmic laser surgical system of claim 3, wherein the plurality of horizontal steps have equal angular sizes, and wherein the offset angles between adjacent horizontal steps are equal to each other throughout the spiral incision and are equal to the angular size of the horizontal steps.

5. The ophthalmic laser surgical system of claim 1, wherein vertical distances between adjacent horizontal steps are equal to each other throughout the spiral incision.

6. The ophthalmic laser surgical system of claim 1, wherein the controller is configured to scan the focal spot of the pulsed laser beam to form a plurality of layers within the lens,
wherein each layer is located at a defined vertical position, and includes a closed curve which is a cross-section of the boundary incision surface and a filled area forming one of the horizontal steps.

7. The ophthalmic laser surgical system of claim 6, wherein the closed curve is a circle, and the filled area is a sector of the circle and is formed by scanning the focal spot along a plurality of radial lines or along a plurality of arcs.

8. The ophthalmic laser surgical system of claim 7, wherein for a given layer *i*, a vertical position *Zᵢ* of the layer *i* is *Zᵢ* = *Z₀ + i*d,* where *Z₀* is a constant and *d* is a vertical distance between adjacent layers, and an angular position *ϕᵢ* of the sector is *ϕᵢ* = *ϕ₀ + i*α,* where *ϕ₀* is a constant and *α* is an offset angle between horizontal steps of adjacent layers.

9. The ophthalmic laser surgical system of claim 6, wherein a vertical distance between adjacent layers (d) is approximately equal to an average spot-to-spot distance of the focal spots within each layer.

10. The ophthalmic laser surgical system of claim 1, wherein the lens segmentation pattern further includes a top incision and/or a bottom incision, and wherein the three-dimensional spiral volume is located in a center portion of the lens.

## Patentansprüche

1. Ophthalmisches Laserchirurgiesystem zur Behandlung des Auges eines Patienten, umfassend:
eine gepulste Laserquelle (4), die konfiguriert ist, um einen gepulsten Laserstrahl (6) zu erzeugen;
ein optisches Abgabesystem, das einen Scanner (50) einschließt und konfiguriert ist, um einen Brennfleck des gepulsten Laserstrahls an das Auge (68) abzugeben; und
eine Steuerung (300), die mit der Laserquelle und dem optischen Abgabesystem verbunden und konfiguriert ist, um die Laserquelle und den Scanner zu steuern, um den Brennfleck des gepulsten Laserstrahls innerhalb einer Linse (500) des Auges gemäß einem Linsensegmentierungsmuster zu scannen, um ein dreidimensionales Spiralvolumen in der Linse zu bilden, einschließlich zum:
Ausbilden einer Begrenzungsinzisionsoberfläche (402), die eine äußere Begrenzung des Linsensegmentierungsmusters definiert; und
Ausbilden einer spiralförmigen Inzisionsoberfläche (401), die innerhalb der äußeren Begrenzung angeordnet ist,
wobei die spiralförmige Inzisionsoberfläche eine Vielzahl von horizontalen Stufen (401A-403C, 401i-401j, 411i-j, 421i-421j, 431i-431j,) einschließt, wobei jede horizontale Stufe ein Bereich ist, der sich an einer definierten vertikalen Position entlang einer vertikalen Achse befindet, wobei zwei beliebige horizontale Stufen, die in ihren vertikalen Positionen benachbart sind, in einer Winkelrichtung um die vertikale Achse um einen Versatzwinkel (α) versetzt sind, und wobei eine Vorderkante jeder horizontalen Stufe bei Betrachtung entlang der vertikalen Achse an einer Hinterkante ihrer benachbarten horizontalen Stufe ausgerichtet ist.

2. Ophthalmisches Laserchirurgiesystem nach Anspruch 1, wobei die Begrenzungsinzisionsoberfläche eine zylindrische Oberfläche ist.

3. Ophthalmisches Laserchirurgiesystem nach Anspruch 2, wobei die Begrenzungsinzisionsoberfläche eine runde zylindrische Oberfläche ist, und wobei jede horizontale Stufe die Form eines Kreissektors aufweist.

4. Ophthalmisches Laserchirurgiesystem nach Anspruch 3, wobei die Vielzahl von horizontalen Stufen gleiche Winkelgrößen aufweist, und wobei die Versatzwinkel zwischen benachbarten horizontalen Stufen über die gesamte spiralförmige Inzision hinweg einander gleich sind und gleich der Winkelgröße der horizontalen Stufen sind.

5. Ophthalmisches Laserchirurgiesystem nach Anspruch 1, wobei vertikale Abstände zwischen benachbarten horizontalen Stufen über die gesamte spiralförmige Inzision hinweg einander gleich sind.

6. Ophthalmisches Laserchirurgiesystem nach Anspruch 1, wobei die Steuerung konfiguriert ist, um den Brennfleck des gepulsten Laserstrahls zu scannen, um eine Vielzahl von Schichten innerhalb der Linse zu bilden,
wobei jede Schicht an einer definierten vertikalen Position angeordnet ist und eine geschlossene Kurve, die ein Querschnitt der Begrenzungsinzisionsoberfläche ist, und einen gefüllten Bereich, der eine der horizontalen Stufen bildet, einschließt.

7. Ophthalmisches Laserchirurgiesystem nach Anspruch 6, wobei die geschlossene Kurve ein Kreis ist und der gefüllte Bereich ein Sektor des Kreises ist und durch Abtasten des Brennflecks entlang einer Vielzahl von radialen Linien oder entlang einer Vielzahl von Bögen gebildet wird.

8. Ophthalmisches Laserchirurgiesystem nach Anspruch 7, wobei für eine gegebene Schicht *i* eine vertikale Position *Zᵢ* der Schicht *i Zᵢ = Z₀ + i*d,* ist, wobei *Z₀* eine Konstante ist und d ein vertikaler Abstand zwischen benachbarten Schichten ist, und eine Winkelposition *ϕᵢ* des Sektors *ϕᵢ* = *ϕ₀ + i*α,* ist, wobei *ϕ₀* eine Konstante ist und α ein Versatzwinkel zwischen horizontalen Stufen benachbarter Schichten ist.

9. Ophthalmisches Laserchirurgiesystem nach Anspruch 6, wobei ein vertikaler Abstand zwischen benachbarten Schichten (d) etwa gleich einem durchschnittlichen Punkt-zu-Punkt-Abstand der Brennflecken innerhalb jeder Schicht ist.

10. Ophthalmisches Laserchirurgiesystem nach Anspruch 1, wobei das Linsensegmentierungsmuster ferner eine obere Inzision und/oder eine untere Inzision umfasst, und wobei das dreidimensionale Spiralvolumen in einem zentralen Abschnitt der Linse angeordnet ist.

## Revendications

1. Système chirurgical au laser ophtalmique destiné à traiter un œil d'un patient, comprenant :
une source laser pulsée (4) configurée pour générer un faisceau laser pulsé (6) ;
un système de distribution optique comportant un dispositif de balayage (50), configuré pour distribuer un point focal du faisceau laser pulsé à l'œil (68) ; et
un dispositif de commande (300) connecté à la source laser et au système de distribution optique, configuré pour commander la source laser et le dispositif de balayage pour balayer le point focal du faisceau laser pulsé à l'intérieur d'une lentille (500) de l'œil selon un modèle de segmentation de lentille pour former un volume en spirale tridimensionnelle dans la lentille, y compris pour :
former une surface d'incision de limite (402) définissant une limite externe du motif de segmentation de lentille ; et
former une surface d'incision en spirale (401) située à l'intérieur de la limite extérieure,
dans lequel la surface d'incision en spirale comporte une pluralité de marches horizontales (401A-403C, 401i-401j, 411i-j, 421i-421j, 431i-431j,), chaque marche horizontale étant une zone située au niveau d'une position verticale définie le long d'un axe vertical, dans lequel deux marches horizontales quelconques qui sont adjacentes dans leurs positions verticales sont décalées dans une direction angulaire autour de l'axe vertical d'un angle de décalage (α), et dans lequel un bord d'attaque de chaque marche horizontale est aligné sur un bord de fuite de sa marche horizontale adjacente lorsqu'il est observé le long de l'axe vertical.

2. Système chirurgical au laser ophtalmique selon la revendication 1, dans lequel la surface d'incision de limite est une surface cylindrique.

3. Système chirurgical au laser ophtalmique selon la revendication 2, dans lequel la surface d'incision de limite est une surface cylindrique ronde, et dans lequel chaque marche horizontale a une forme d'un secteur d'un cercle.

4. Système chirurgical au laser ophtalmique selon la revendication 3, dans lequel la pluralité de marches horizontales ont des tailles angulaires égales, et dans lequel les angles décalés entre les marches horizontales adjacentes sont égaux les uns aux autres tout au long de l'incision en spirale et sont égaux à la taille angulaire des marches horizontales.

5. Système chirurgical au laser ophtalmique selon la revendication 1, dans lequel des distances verticales entre des marches horizontales adjacentes sont égales les unes aux autres tout au long de l'incision en spirale.

6. Système chirurgical au laser ophtalmique selon la revendication 1, dans lequel le dispositif de commande est configuré pour balayer le point focal du faisceau laser pulsé afin de former une pluralité de couches à l'intérieur de la lentille,
dans lequel chaque couche est située au niveau d'une position verticale définie, et comporte une courbe fermée qui est une section transversale de la surface d'incision de limite et une zone remplie formant l'une des marches horizontales.

7. Système chirurgical au laser ophtalmique selon la revendication 6, dans lequel la courbe fermée est un cercle, et la zone remplie est un secteur du cercle et est formée par balayage du point focal le long d'une pluralité de lignes radiales ou le long d'une pluralité d'arcs.

8. Système chirurgical au laser ophtalmique selon la revendication 7, dans lequel pour une couche donnée *i,* une position verticale *Zᵢ* de la couche *i* est *Zᵢ = Z₀ + i * d,* où *Z₀* est une constante et d est une distance verticale entre des couches adjacentes, et une position angulaire *ϕᵢ* du secteur est *ϕᵢ* = *ϕ₀ + i * α,* où *ϕ₀* est une constante et α est un angle de décalage entre des marches horizontales des couches adjacentes.

9. Système chirurgical au laser ophtalmique selon la revendication 6, dans lequel une distance verticale entre des couches adjacentes (d) est approximativement égale à une distance moyenne entre points des points focaux à l'intérieur de chaque couche.

10. Système chirurgical au laser ophtalmique selon la revendication 1, dans lequel le motif de segmentation de lentille comporte en outre une incision supérieure et/ou une incision inférieure, et dans lequel le volume en spirale tridimensionnel est situé dans une partie centrale de la lentille.
